# EUROPÄISCHE PATENTSCHRIFT

(11) **EP 2 460 476 B1**
(45) Veröffentlichungstag und Bekanntmachung des Hinweises auf die Patenterteilung: **25.11.2020**
(21) Anmeldenummer: 11191896.7
(22) Anmeldetag: 16.04.2007
(51) Int. Cl.: A61B 17/00, A61B 17/12, A61F 2/01

(54) **Occluder zum Verschließen eines Herzohres und Herstellungsverfahren dafür**
Occluder for closing a cardiac auricle and manufacturing method therefor
Dispositif d'occlusion destiné à fermer une oreillette cardiaque et son procédé de fabrication

(43) Veröffentlichungstag der Anmeldung: 06.06.2012
(62) Teilanmeldung aus: 07106278.0
(73) Patentinhaber: Occlutech Holding AG, 8200 Schaffhausen (CH)
(72) Erfinder: Figulla, Prof. Hans-Reiner, 07749 Jena (DE); Schräder, Prof. Dr. med Rainer, 60596 Frankfurt (DE); Krizanic, Dr. Florian, 07743 Jena (DE); Moszner, Dr. Robert, 07639 Bad Klosterlausnitz (DE); Moszner, Friedrich, 99441 Hohlstedt (DE); Schmidt, Dr. Kathrin, 07768 Kahla (DE); Ottma, Rüdiger, 99441 Großschwabhausen (DE)
(74) Vertreter: KIPA AB

(56) Entgegenhaltungen:
- WO-A-02/071977
- WO-A1-2007/110195
- US-A- 5 944 738
- US-A1- 2005 113 861
- US-A1- 2006 224 183
- US-A1- 2007 043 391
- US-B1- 6 168 622

## Beschreibung

### Gebiet der Erfindung

Die vorliegende Erfindung betrifft ein selbst expandierbares Occlusionsinstrument zum Verschließen eines Herzohres, bestehend aus einem Geflecht dünner Drähte, oder Fäden, welches mittels eines Formgebungs-, Umformungs- und/oder Wärmebehandlungsverfahrens eine geeignete Formgebung erhält, wobei das Occlusionsinstrument einen vorderseitigen proximalen Retentionsbereich und einen rückseitigen distalen Retentionsbereich aufweist, und wobei in dem proximalen Retentionsbereich die Enden der Drähte oder Fäden in einer Fassung aber auch in einer zweiten Lösung ohne Fassung zusammenlaufen. Des Weiteren weist das Occlusionsinstrument einen Mittenbereich zwischen dem proximalen und dem distalen Retentionsbereich auf. Das Occlusionsinstrument ist dabei so ausgeführt, dass es in einem zusammengefalteten Zustand mittels eines Katheters in den Körper eines Patienten minimal-invasiv einführbar und in dem Herzohr des Patienten positionierbar ist. Des Weiteren betrifft die Erfindung Verfahren zur Herstellung eines solchen Occlusionsinstruments.

### Hintergrund der Erfindung

Ein derartiges Occlusionsinstrument ist zumindest teilweise dem Prinzip nach aus der Medizintechnik bekannt. Beispielsweise ist in der DE 10 338 702 vom 22. August 2003 ein Occlusionsinstrument zur Behandlung von Septumdefekten bekannt, welches aus einem Geflecht dünner Drähte bzw. Fäden besteht und mittels eines Umformungs- und Wärmebehandlungsverfahrens eine geeignete Formgebung erhält. Das bekannte Occlusionsinstrument weist einen distalen Retentionsbereich, welcher besonders flach ausgeprägt ist, einen proximalen Retentionsbereich und einen zylindrischen Steg zwischen dem proximalen und dem distalen Retentionsbereich auf. Am proximalen Retentionsbereich laufen die Enden der das Geflecht bildenden Drähte in einer Fassung zusammen. Dabei ist vorgesehen, dass die beiden Retentionsbereiche des bekannten Occlusionsinstruments durch einen meist intravaskulären Operationseingriff beiderseits eines zu verschließenden Shuntes in einem Septum zur Anlage kommen, während der Steg durch den Shunt hindurchläuft. Das Occlusionsinstrument ist angepasst, um eine Öffnung mit dynamischem Blutfluss permanent zu verschließen, und weist dazu eine spezielle Konstruktion auf und ist nicht für Herzohrocclussionen geeignet.

Ähnliche Instrumente sind in der Occlutech-Patentanmeldung Deutschland Nr. 102005053957.2 vom 11.05.2005 und die gleich lautenden Anmeldungen PCT/EP2005/012130 vom 11.05.2005 und die US-Anmeldung US11/271,751 vom 19.12.2005 beschrieben.

Wenn der Patient unter einem so genannten Vorhofflimmern des Herzens leidet, treten besondere emboliebedingte Probleme in Erscheinung. Hierbei handelt es sich um eine frequente Erregung der Vorhöfe des Herzens, die zu keiner Kontraktion der Vorhöfe führt. Folge dieses Kontraktionsverlustes der Vorkammern des Herzens ist es, dass eine wirksame Durchwirbelung und Durchmischung des Blutes ausbleibt und sich Tromben im Vorhof bilden können. Ein erhebliches Risiko bei der Vorhoftrombenbildung infolge Vorhofflimmerns besteht darin, dass solche Tromben mit dem Blutstrom mitgerissen werden können und in die arterielle Zirkulation gelangen. Folgen dieser Embolisation sind insbesondere Schlaganfälle, die in etwa 5% pro Jahr bei Patienten mit Vorhofflimmern auftreten, falls nicht durch eine chronische Behandlung eine Geringungshemmung des Blutes mit so genannten Dicumerolen durchgeführt wird. Eine Herbeiführung der Geringungshemmung des Blutes mit so genannten Dicumerolen ist allerdings ebenfalls nicht risikolos. Nebenwirkungen der Behandlungen mit Dicumerolen sind vermehrte Blutungen, sodass Kontraindikationen für diese Behandlung bei ca. 20% der Patienten mit Vorhofflimmern besteht und die Patienten somit aufgrund von einer Risikoabwägung Blutung/Schlaganfall das Risiko eines Schlaganfalls in Kauf genommen wird.

Tromben im Vorhof des Herzens entstehen in überwiegender Mehrzahl in den so genannten Herzohren. Die Herzohren sind Ausstülpungen an den Vorhöfen des menschlichen Herzens. Das rechte Herzohr liegt neben der aufsteigenden Aorta, und das linke Herzohr neben der großen Lungenarterie. Dabei ist das linke Herzohr bei Patienten mit Vorhofflimmern der häufige Entstehungsort für Blutgerinnsel, die zu einem Schlaganfall führen können.

Aufgrund der im Zusammenhang mit der zuvor beschriebenen Vorhoftrombenbildung genannten Risiken und Probleme liegt der vorliegenden Erfindung die Aufgabe zugrunde, ein Occlusionsinstrument anzugeben, mit dem das Herzohr des linken Vorhofs verschlossen werden kann, um eine Trombusbildung mit dem Risiko eines Schlaganfalls erheblich zu reduzieren. Insbesondere soll ein Occlusionsinstrument angegeben werden, mit dem das Schlaganfallrisiko auch bei solchen Patienten reduziert werden kann, bei denen eine Gerinnungshemmung mit Dicumerolen (so genannte Antikoagulation) aufgrund von Blutungsneigungen kontra indiziert ist. Ebenso sollte das Occlusionsinstrument sicher im Herzohr zu verankern sein, ohne sich zu lösen.

Nach dem Stand der Technik existieren verschiedene Lösungen, welche im Wesentlichen Teilgeflechte mit gestängeartigen Bauelementen kombinieren, teilweise werden dabei auch gelaserte Konstruktionen in dieser Kombination eingesetzt, das betrifft solche Offenbarungen wie: EP 1417933A1; US 7,169,164B2; US 2005/0234543A1; US 6,689,150B1; US 6,152,144.

Diese Offenbarungen haben unter anderem den Nachteil, dass für die Implantation relativ große Einführschleusen mit bis zu 14 F Innendurchmesser benötigt werden.

Weitere Offenbarungen wie von Amplatz PCT/US 2005/010551, nach der PLAATO-Methode (PLAATO = Percutaneus Left Atrial Appendage Transcatheter) und Watchman-Occluder beherrschen nicht die korrekte vorgegebene Positionierung. Bei dem System PLAATO besteht die Gefahr der Perforation mit dem Herzbeutel, außerdem wurde PLAATO ebenfalls mit einer 14F-Schleuse implantiert.

Eine weitere Anmeldung von Occlutech betrifft die Offenbarung eines einnabigen Herzohroccluders unter Verwendung eines Fixiermittels zum Ausbilden einer kraftschlüssigen Verbindung zwischen dem Geflecht eines Occlusionskörpers und der Herzohrwandung, PCT/EP 2006/005292 vom 02.06.2006. Dabei ist das Fixiermittel ausgelegt, nach dem Applizieren insbesondere in einer kontrollierten Weise zu einem flexiblen unlöslichen Produkt auszuhärten, um somit eine dauerhafte und feste Verankerung zwischen dem Geflecht des Occlusionskörpers und der Herzohrwandung herzustellen. Offenbarungsgemäß ist vorgesehen, dass der distale Retentionsbereich einen Kugelbereich aufweist, der im expandierten Zustand des Occlusionsinstruments in dem zu verschließenden Herzohr an den Innenwandungen des Herzohres zur Anlage kommt und mit den Innenwandungen des Herzohres eine kraftschlüssige Verbindung bildet, um derart das implantierte und expandierte Occlusionsinstrument im Herzohr zu halten, wobei der proximale Retentionsbereich des Occlusionsinstruments die Öffnung des Herzohres verschließt.

In der WO02/071977 werden implantierbare Vorrichtungen beschrieben, beispielsweise bestehend aus einer geflochtenen Grundstruktur mit daran befestigter proximaler Abdeckstruktur.

In der US6,168,622 Patentschrift wird ein geflochtenes Occlusioninstrument für Aneurysmen beschrieben mit jeweils einer Klemme am distalen und proximalen Ende der Vorrichtung.

In der Patentschriften US5,944,738 werden septale Occlusionsinstrumente beschrieben, welche nicht zur sicheren Verankerung in einem Herzohr geeignet sind. Die Vorrichtungen habe auch Klemmen an den beiden Enden der Vorrichtungen.

In der WO2007/110195A1, welche eine Veröffentlichung unter Art. 54(3) EPC ist, werden geflochtene Occlusionsinstrumente beschrieben, jedoch nicht für die sichere Verankerung in einem Herzohr.

### Zusammenfassung der Erfindung

Die vorliegende Erfindung ist alleine in den beigefügten Patentansprüchen definiert, insbesondere in dem unabhängigen Patentanspruch 1. Bezugnahme auf "Ausführungsformen" in der vorliegenden Beschreibung, welche nicht unter den Umfang der Patentansprüche fallen, stellen nur mögliche beispielhafte Ausführungen dar und sind somit nicht Teil der vorliegenden Erfindung.

Aufgabe der Erfindung ist es, die oben genannten Nachteile der herkömmlichen Verfahren und Vorrichtungen zu überwinden und eventuell eine möglichst Kosten senkende und patientenfreundliche sowie patientensichere Lösung anzugeben.

Die oben genannte Aufgabe wird mit einem selbst expandierbaren Occlusionsinstrument zum Verschließen eines Herzohres gemäß Anspruch 1 gelöst, wobei das Occlusionsinstrument aus einem Geflecht dünner Drähte, Litzen oder Fäden besteht, welches mittels eines Formgebungs-, Umformungs- und/oder Wärmebehandlungsverfahrens eine geeignete Formgebung erhalten hat. Dabei ist vorgesehen, dass das Occlusionsinstrument einen vorderseitigen proximalen Retentionsbereich und einen hinterseitigen distalen Retentionsbereich sowie einen zwischen dem proximalen und dem distalen Retentionsbereich angeordneten Mittenbereich aufweist, und wobei das Occlusionsinstrument im zusammengefalteten Zustand mittels eines Katheters in den Körper eines Patienten minimal-invasiv einführbar und in dem Herzohr des Patienten positionierbar ist.

Bevorzugte Weiterentwicklungen der Erfindung bezüglich des Occlusionsinstruments sind in den Unteransprüchen angegeben.

### Kurzbeschreibung der Zeichnungen

Im Folgenden werden bevorzugte Ausführungsbeispiele für Occlusionsinstrumente anhand der Zeichnungen näher erläutert. Es zeigen:
Fig. 1 Eine Seitenansicht einer Ausführungsform eines Herzohroccluders mit einer Fassung;
Fig. 2 Die Ausführungsform des Herzohroccluders nach Fig. 1 nach Implantation im linken Herzohr (Englisch Left Atrial Appendage - LAA), angrenzend zum linken Vorhof;
Fig. 3, 4 Kugelgeflecht, kugel- und ballonförmig zur Herstellung eines Herzohroccluders nach Fig. 1;
Fig. 5 Eine Seitenansicht einer Ausführungsform eines Herzohroccluders ohne Fassung;
Fig. 6 Die Ausführungsform nach Fig. 5 im Anschluss an eine Implantation im linken Herzohr des linken Vorhofes;
Fig. 7 Kugelgeflecht aus einem Drahtstück ohne Fassung;
Fig. 8 Seitenansicht einer zweiten Variante einer Ausführungsform eines Herzohroccluders mit einer Fassung;
Fig. 9 Die Ausführungsform nach Fig. 8 nach Implantation im linken Herzohr;
Fig. 10 Dritte Variante einer Ausführungsform des Herzohroccluders in einer Seitenansicht;
Fig. 11 Darstellung einer flexiblen Handhabung des Herzohroccluders nach der dritten Variante;
Fig. 12 Darstellung eines Herzohroccluders nach der dritten Variante mit aufgenähtem Patch am proximalen Ende;
Fig. 13 Darstellung eines Herzohroccluders mit eingenähtem Patch im proximalen Ende;
Fig. 14 Darstellung einer vierten Variante einer Ausführungsform eines Herzohroccluders mit langem Steg zwischen proximalem und distalem Ende;
Fig. 15 Fünfte Variante einer Ausführungsform eines Herzohroccluders mit einer flachen Doppelscheibe am proximalen Ende;
Fig. 16 Die Ausführungsform nach der fünften Variante im Anschluss der Implantation in das linke Herzohr;
Fig. 17 Sechste Variante einer Ausführungsform nach der Implantation im linken Herzohr;
Fig. 18 Siebte Variante einer Ausführungsform eines Herzohroccluders 180, hergestellt aus einem Trichtergeflecht am distalen Ende 21.
Fig. 19 Darstellung einer Seitenansicht eines PFO-Occluders 190 ohne Fassung, hergestellt aus einem Kugelgeflecht nach Fig. 7.
Fig. 20 Darstellung einer Seitenansicht eines ASD-Occluders 200 ohne Fassung, hergestellt aus einem Kugelgeflecht nach Fig. 7.
Fig. 21 Darstellung eines Trichtergeflechts 210, hergestellt aus einem Draht nach dem 2-D-Flechtverfahren oder Flechtverfahren nach Occlutech.
Fig. 22 Seitenansicht eines PFO-Occluders 220 ohne Fassung, hergestellt aus einem Draht nach dem Trichtergeflecht gemäß Fig. 21.
Fig. 23 Seitenansicht eines ASD-Occluders 230 ohne Fassung, hergestellt aus einem Draht nach dem Trichtergeflecht Fig. 21; und
Fig. 24 Darstellung eines Herzohres.

### Ausführliche Beschreibung

Während die Erfindung nachstehend in verschiedenen Ausführungsformen zum Durchführen der Erfindung beschrieben werden wird, versteht der Fachmann, dass diese Ausführungsformen nur illustrative, nicht begrenzende Beispiele von vielfältigen Formen, welche die vorliegende Erfindung einnehmen kann, darstellen.

Selbstverständlich sind hier aber auch andere, wie auch immer geartete und anwendungsspezifische Formgebungen des Occlusionsinstrumtents denkbar. Die hier dargestellten Formgebungen dienen lediglich zur Beschreibung von bevorzugten Ausführungsformen des Occlusionsinstruments und soll insbesondere nicht den Schutzumfang der Erfindung in irgendeiner Weise einschränken.

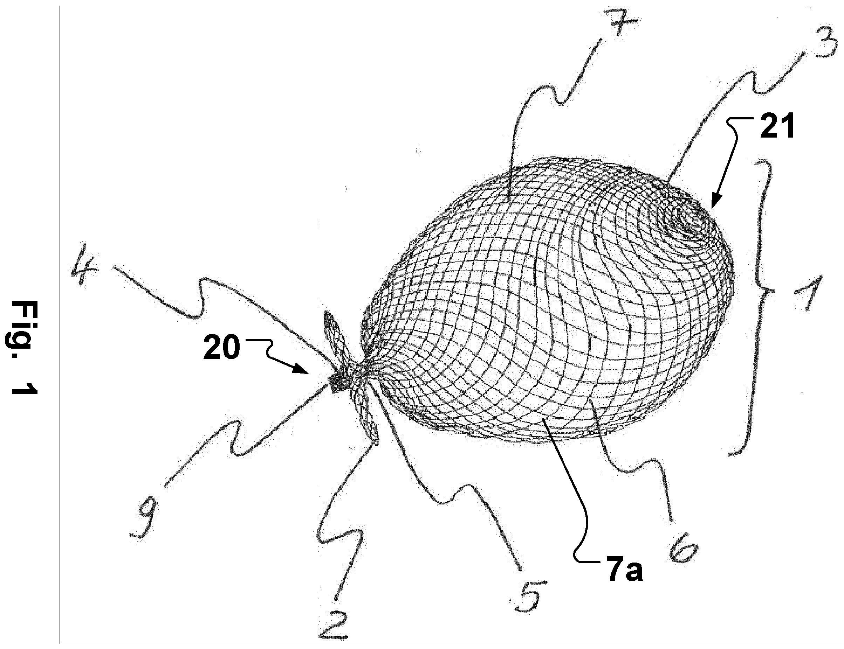

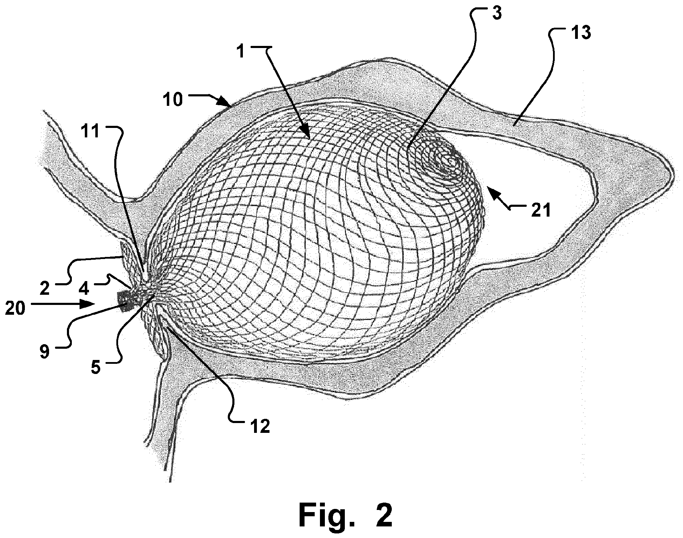

Fig. 1 zeigt eine Seitenansicht einer Ausführungsform des selbst expandierbaren Occlusionsinstruments. In der Fig. 2 sehen wir eine Schnittdarstellung im Bereich linker Vorhof/linkes Herzohr mit implantiertem Herzohroccluder in der bevorzugten Ausführungsform mit einer proximalen Fassung.

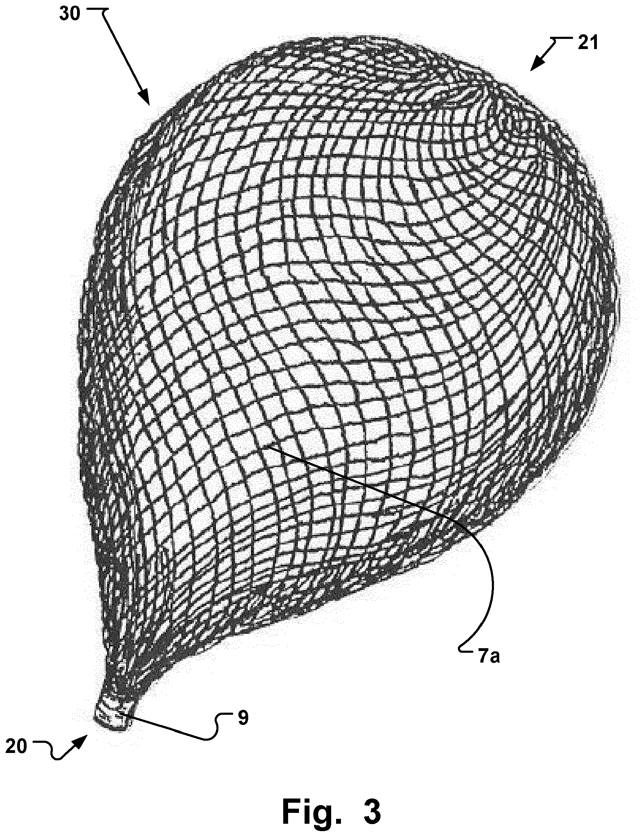

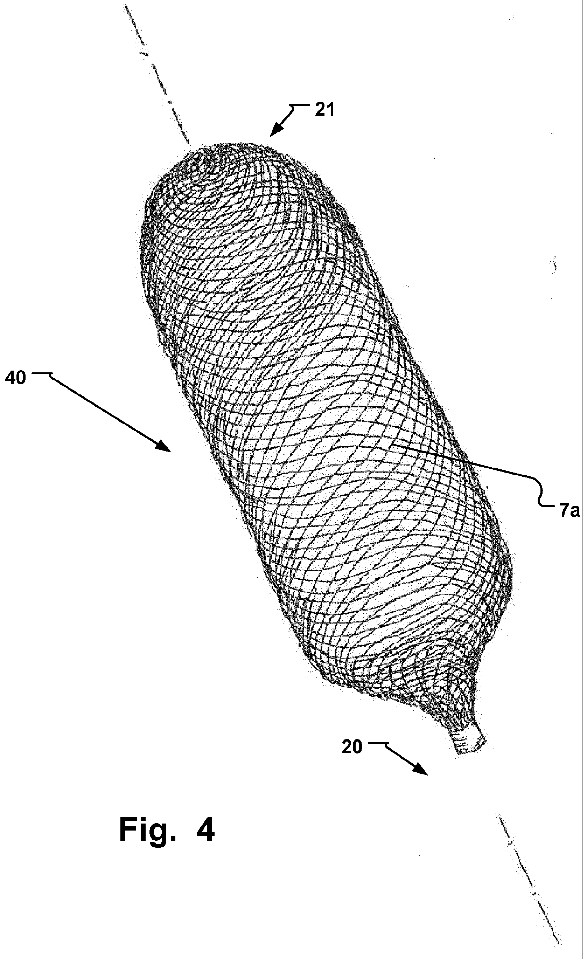

Das Occlusionsinstrument 1 der in Fig. 1 und Fig. 2 dargestellten, bevorzugten Ausführungsform besteht aus einem Geflecht dünner Drähte oder Fäden, das mit einem Umformungs- und/oder Wärmebehandlungsverfahren eine geeignete Formgebung erhält. Dazu werden nach Fig. 3 und 4 entsprechende Kugel-Drahtgeflechte mit einer proximalen Fassung verwendet.

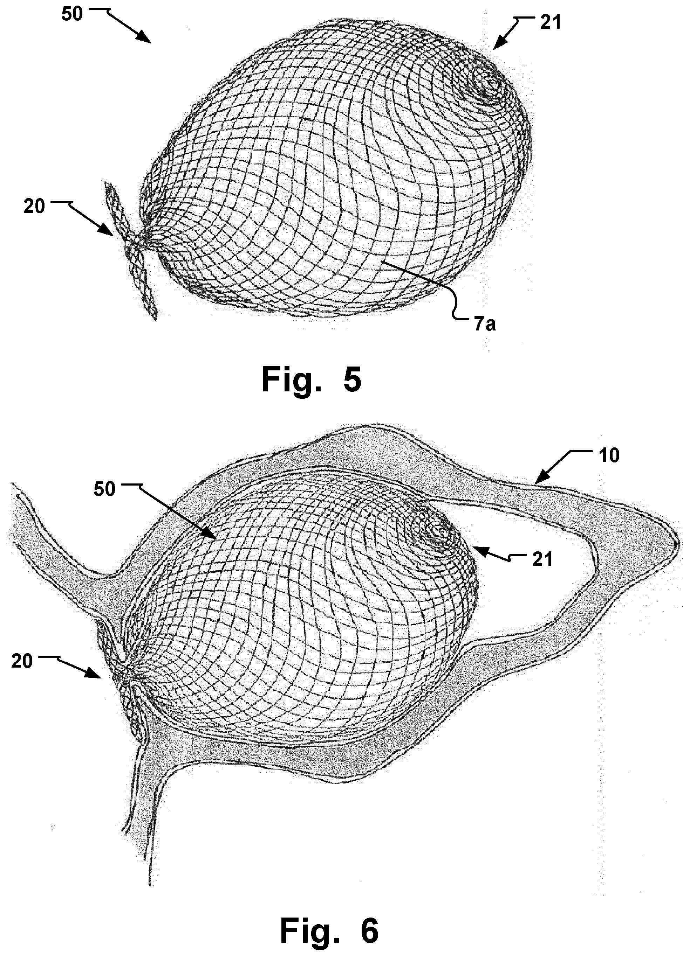

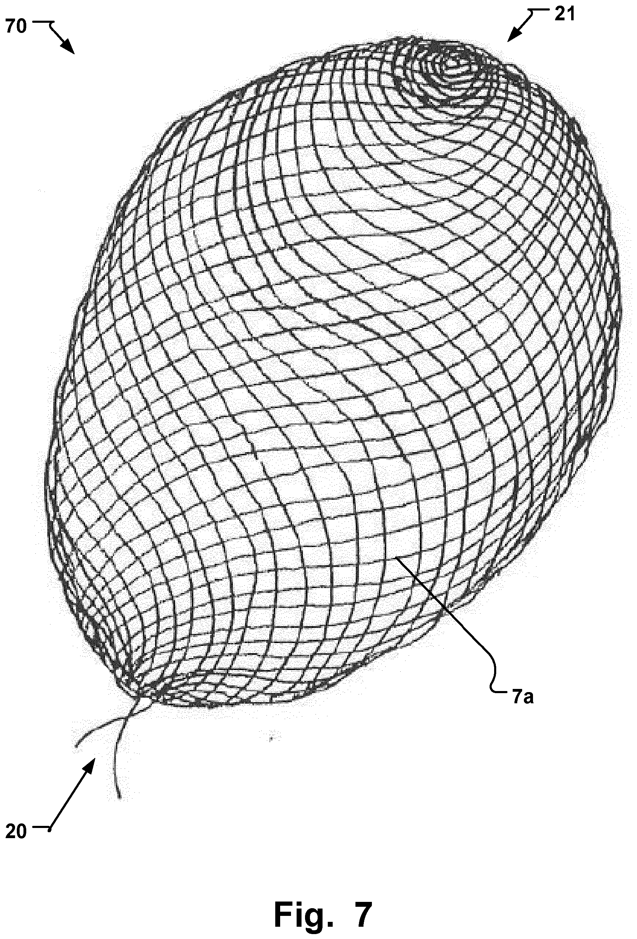

Fig. 5 und 6 zeigen eine Ausführungsform eines selbst expandierbaren Occlusionsinstruments ohne Fassung bestehend aus einem Geflecht, wie vorstehend beschrieben, aus einem Drahtstück geflochten zu einem Kugelgebinde nach Fig. 7 und mit einem Umformungs- und/oder Wärmebehandlungsverfahren in die gewünschte Endform gebracht.

Bei dem Occlusionsinstrument 1 handelt es sich um einen Herzohroccluder mit einer Formgebung bestehend aus einem vorderseitigen proximalen Retentionsbereich 2, einem Mittenbereich 5 und einem rückseitigen, kugelförmigen distalen Retentionsbereich 3. In dem proximalen Retentionsbereich 2 laufen die Enden der Drähte bzw. Fäden des Geflechts 7 in einer Fassung 4 zusammen. Der distale Retentionsbereich 3 hingegen weist in der bevorzugten Form ein geschlossenes Ende ohne Fassung aus.

Aufgrund der Form ähnlich einer Erdbeere wird das Occlusionsinstrument auch "Strawberry-Occluder" genannt.

Das Geflecht 7 ist aus Drähten oder Fäden gebildet, welche in bevorzugter Weise aus Nitinol oder aus einem anderen Material mit Formgedächtnis oder Memory-Effekt bestehen. Denkbar hierbei wäre es, ein Polymerkunststoff einzusetzen, der die Formgedächtniseigenschaft aufweist. Auch ist es denkbar, ein biologisch abbaubares Formgedächtnismaterial zu verwenden. Wesentlich ist, dass das Geflecht 7 eine ausreichende Flexibilität aufweist, sodass das Occlusionsinstrument 1 auf den Durchmesser eines bei einem minimal-invasiven, insbesondere intravaskulären Operationseingriff verwendeten (nicht explizit dargestellten) Katheters verjüngt werden kann. Aufgrund des Memory-Effekts des Materials verfügt das derart verjüngte Occlusionsinstrument 1 eine Formgedächtnisfunktion, sodass sich das Instrument 1 nach dem Verlassen des Katheters selbstständig expandiert und eine der Verwendung entsprechende, vorbestimmte Form wieder einnimmt. Üblicherweise erfolgt dies, nachdem das zunächst im Katheter angeordnete Occlusionsinstrument 1 an der zu behandelnden Stelle, insbesondere im Herzohr des Herzens eines Patienten, platziert wurde.

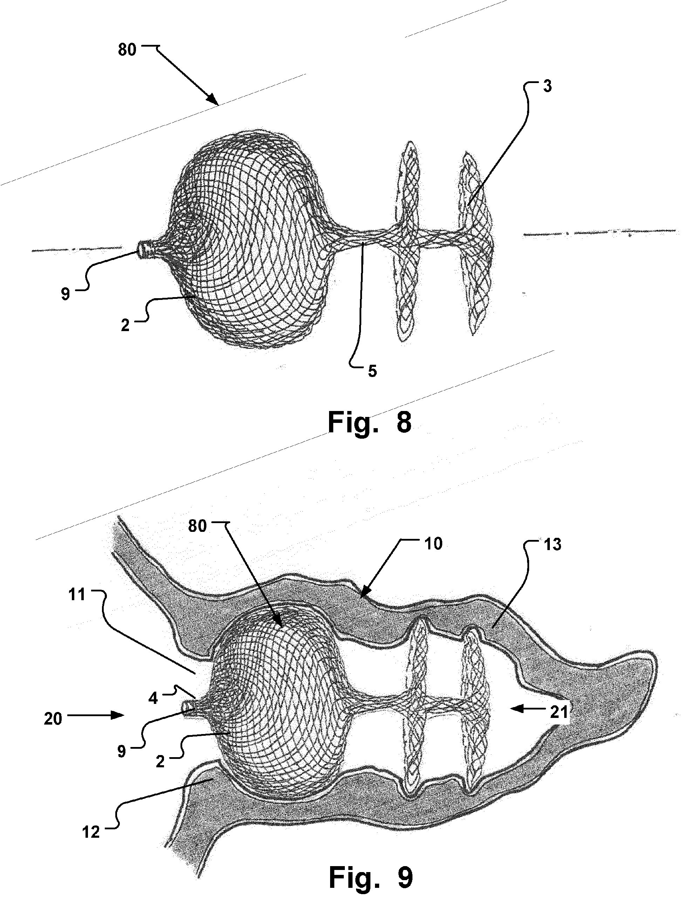

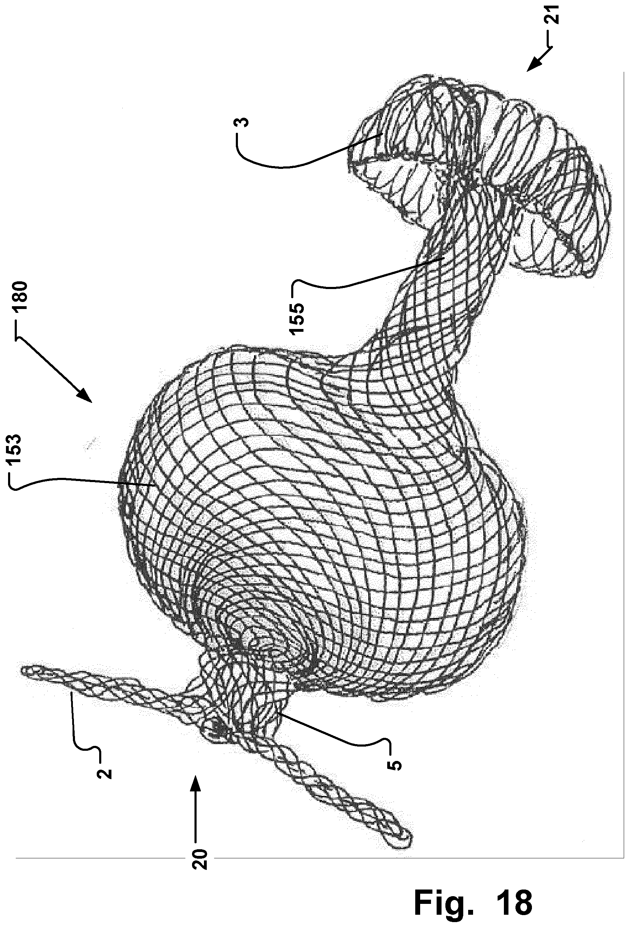

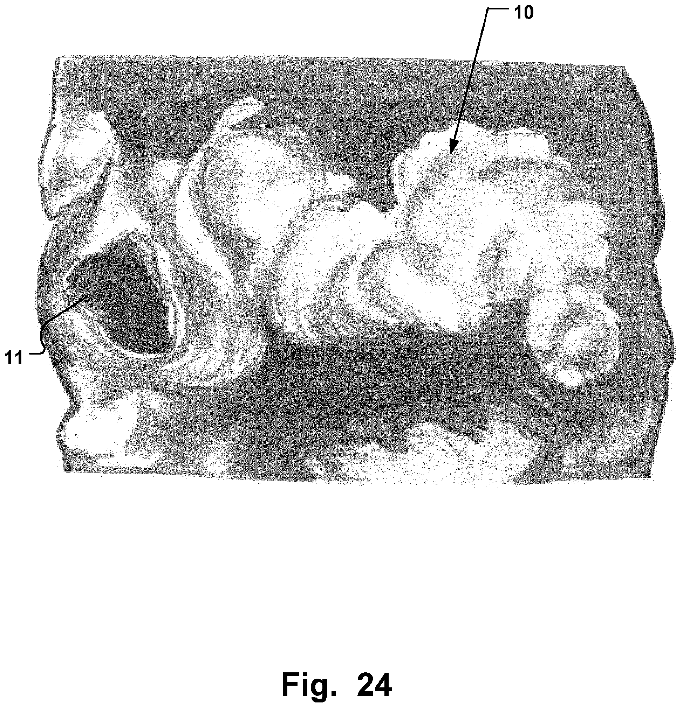

Die vorbestimmte Form des Occlusionsinstruments kann auch eine glockenähnliche Form sein, wobei das sich das verjüngende Ende der glockenähnlichen Form den distalen Retentionsbereich 3 bildet, wie in Fig. 18 dargestellt. Fig. 8 und 9 bzw. 10 bis 17 stellen verschiedene Varianten des Herzohroccluders in der bevorzugten Variante nach Fig. 1 und 2 dar und können durchaus ihre Anwendung finden auf Grund der hohen Variationsbreite und Spezifik des Herzohres, wie in Fig. 24 dargestellt.

In den Figuren 1, 2, 5 und 6 sind Occlusionsinstrumente im expandierten Zustand dargestellt. Wie bereits angedeutet, weist das Occlusionsinstrument 1 einen proximalen Retentionsbereich 2, einen distalen Retentionsbereich 3 sowie einen taillierten, zylindrischen Mittenbereich 5 auf. Der Herzohroccluder weist ein proximales Ende 20 sowie ein distales Ende 21 auf. Der distale Retentionsbereich 3 mit dem daran ausgebildeten Kugelbereich 6 dient in erster Linie zum Befestigen und Halten des implantierten und expandierten Occlusionsinstruments 1 im Herzohr eines Patienten. Hierzu ist vorgesehen, dass der Kugelbereich 6 in dem zu verschließenden Herzohr an den Innenwandungen des Herzohres zur Anlage kommt und mit den Innenwandungen des Herzohres eine kraftschlüssige Verbindung bildet und somit das implantierte und expandierte Occlusionsinstrument 1 in dem Herzohr hält. Denkbar wäre es beispielsweise, dass der distale Retentionsbereich 3 bzw. der Kugelbereich 6 unter einer radialen Vorspannung stehen, sodass damit für eine relativ breite Variation von Herzohröffnungen der sichere Halt des expandierten Occlusionsinstruments 1 garantiert werden kann.

Im implantierten und expandierten Zustand dient der proximale Retentionsbereich 2 dazu, die Öffnung des Herzohres möglichst optimal zu verschließen. Auf die Einzelheiten der Funktionsweise der einzelnen Retentionsbereiche wird unter Bezugnahme auf die Fig. 2 und 6 im Nachfolgenden näher eingegangen.

Der Konstruktion des Occlusionsinstruments 1 liegt das Prinzip zugrunde, dass sich das Occlusionsinstrument 1 auf die Größe eines Katheters verjüngen lässt. Nach dem Austritt aus dem Katheter entfalten sich dann die Retentionsbereiche 3, 2 selbstständig und legen sich an die Innenwandungen des Herzohres an. Bei der offenbarten Konstruktion handelt es sich somit um ein in gewissen Maßen selbstpositionierendes und selbstzentrierendes System. Der Mittenbereich 5 hat dabei eine für die Anwendung vorab festgelegte Länge, um das Verschließen der Herzohröffnung zu gewährleisten.

Durch die flexible Eigenschaft des erfindungsgemäßen Occlusionsinstruments 1 aufgrund der verwendeten Materialien und aufgrund des Geflechtes 7 ist das Instrument 1 reversibel zusammen- und auseinanderfaltbar ausgeführt, sodass ein bereits expandiertes Occlusionsinstrument 1 beispielsweise mithilfe eines Explantations- Katheters zusammenfaltbar ist, wobei die kraftschüssige Verbindung zwischen dem Kugelbereich 6 und den Innenwandungen des Herzohres 10 dann gelöst werden.

Fig. 2 und 6 zeigen das Occlusionsinstrument 1 im implantierten Zustand. Im Einzelnen ist das Occlusionsinstrument im linken Herzohr 10 des Herzens eines Patienten eingesetzt und dient zum Verschließen des Herzohres. Im Einzelnen liegt der Kugelbereich 6 des distalen Retentionsbereiches 3 an den Innenwandungen des Herzohres 10 an und dient zum Positionieren und Fixieren des implantierten Occlusionsinstrumentes 1. Im implantierten Zustand schließt der proximale Retentionsbereich 2 mit der Öffnung des Herzohres 10 ab, wobei die Peripherie des distalen Retentionsbereiches an der Wandung 12 der Herzohröffnung zur Anlage kommt, während der Mittenbereich 5 durch die Öffnung 11 hindurchläuft. Das Occlusionsinstrument 1 stellt von daher ein Verschlusssystem dar, dass mittels minimal-invasiver Verfahren, d.h. beispielsweise über einen Katheter und Führungsdrähte, dem Körper eines Patienten zugeführt und an der dafür bestimmten Stelle positioniert wird. Dabei ist insbesondere der proximale Retentionsbereich 2 des Instruments 1 derart ausgebildet, dass keinerlei Material des implantierten Occlusionsinstruments 1 über die Herzohrwand in den Blutstrom des Patienten gelangen kann. Der Rand des proximalen Retentionsbereiches 2 schließt dabei bündig mit der Herzohrwand 13 ab. Dies erfolgt über einen relativ weiten Bereich unabhängig von dem Herzohrdurchmesser und der Stärke der Herzohrwandung 12 an der Herzohröffnung 11. Dadurch kann erreicht werden, dass nach dem Implantieren des Occlusionsinstruments 1 relativ schnell eine vollständige Endothelialisierung eintritt und mögliche Abwehrreaktionen vom Körper des Patienten nicht eintreten, da ein Blutkontakt mit dem Werkstoff des Implantats 1 wirksam verhindert wird.

Fig. 3 zeigt ein kugelförmiges hohles Kugelgeflecht 30 aus Fäden 7a auf, zur Herstellung eines Herzohroccluders.

Fig. 4 ein ballonförmiges hohles Kugelgeflecht 40 aus Fäden 7a auf, zur Herstellung eines Herzohroccluders.

In Fig. 5 ist in einer Seitenansicht einer Ausführungsform eines Herzohroccluders 50 ohne Fassung gezeigt. Der Herzohroccluder 50 weist am proximalen Ende keine Fassung auf. In Fig. 6 ist die Ausführungsform des Herzohroccluders 50 nach Fig. 5 im Anschluss an eine Implantation im linken Herzohr 10 des linken Vorhofes gezeigt.

Fig. 7 illustriert ein Kugelgeflecht 70 aus einem Drahtstück ohne Fassung.

Fig. 8 zeigt in einer Seitenansicht einer zweite Variante einer Ausführungsform eines Herzohroccluders 80 mit einer Fassung. Fig. 9 Die Ausführungsform nach Fig. 8 nach Implantation im linken Herzohr 10;

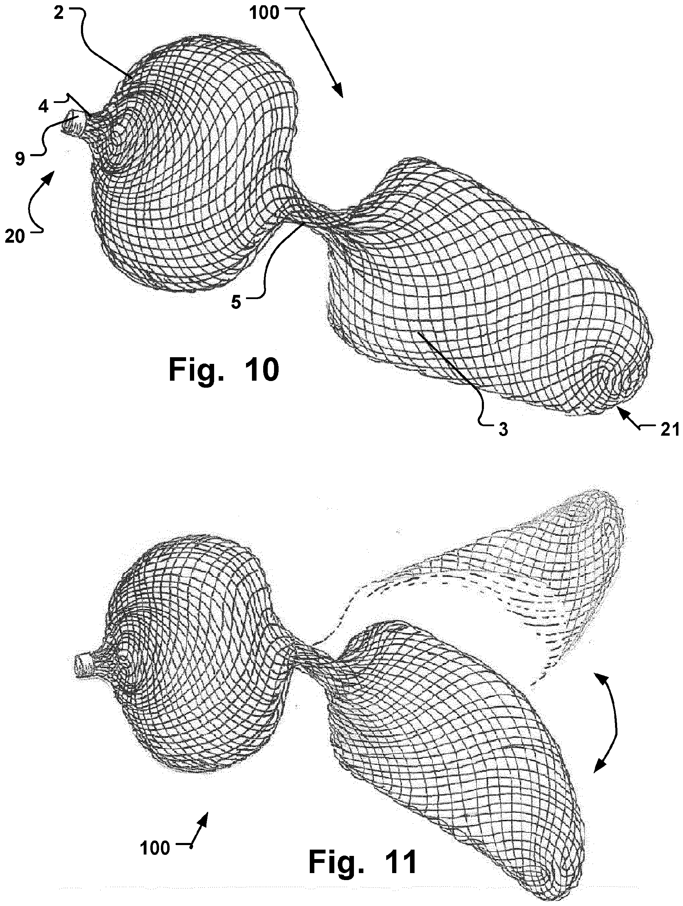

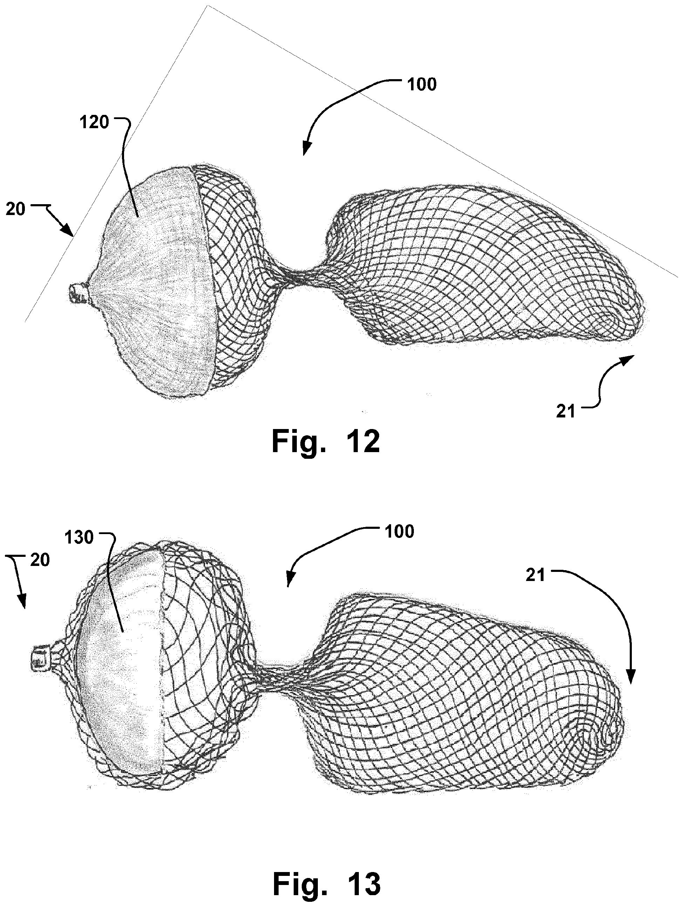

Fig. 10 illustriert eine dritte Variante einer Ausführungsform des Herzohroccluders 100 in einer Seitenansicht. Fig. 11 Darstellung einer flexiblen Handhabung des Herzohroccluders 100 nach der dritten Variante. Fig. 12 ist eine Darstellung eines Herzohroccluders 100 nach der dritten Variante mit aufgenähtem Patch 120 am proximalen Ende. Fig. 13 Darstellung eines Herzohroccluders mit eingenähtem Patch 130 im proximalen Ende. Wie in den Figuren 12 und 13 dargestellt, kann das Occlusionsinstrument gemäß gewissen Ausführungsformen des Weiteren Gewebeeinlagen 120 bzw. 130 aufweisen, wobei diese beispielsweise aus dem Material Dacron (Polyethylenterephthalat) bestehen können. Denkbar ist dabei die Gewebeeinlagen im Inneren oder auf dem proximalen Retentionsbereich 2 einzuarbeiten, um die Herzohröffnung vollständig verschließen zu können. Die Einlagerung der Gewebeeinlagen kann z.B. durch Verspannung dieser im Occlusionsinstrument 1 erfolgen. Das im Körper eingesetzte Implantat wird dann bereits nach einigen Wochen bzw. Monaten vollständig von körpereigenem Gewebe eingeschlossen. Somit erfolgt ein vorteilafter Verschluss des Herzohres 10.

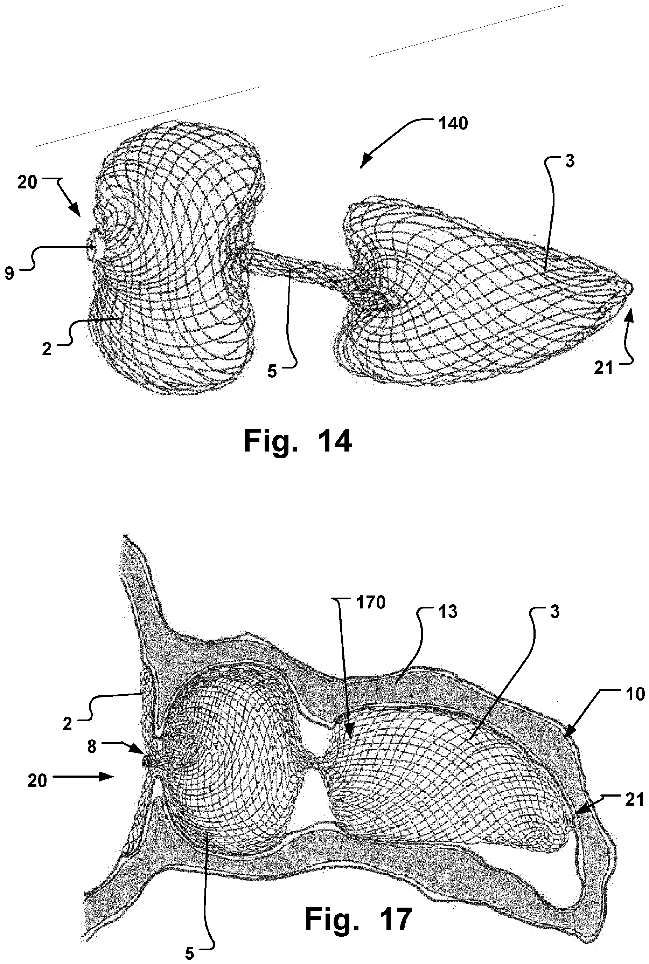

Fig. 14 ist eine Darstellung einer vierten Variante einer Ausführungsform eines Herzohroccluders 140 mit langem Steg zwischen proximalem Ende 20 und distalem Ende 21.

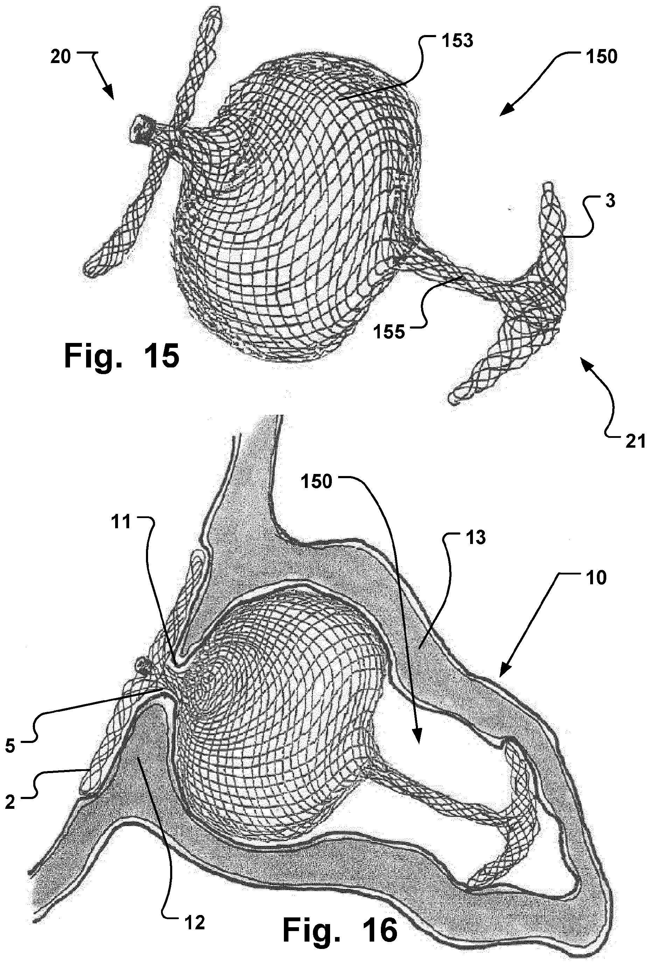

Fig. 15 zeigt eine fünfte Variante einer Ausführungsform eines Herzohroccluders 150 mit einer flachen Doppelscheibe am proximalen Ende 20 und einem Zwischen-Retentionsbereich 151. In Fig. 16 wird die Ausführungsform nach der fünften Variante im Anschluss der Implantation in das linke Herzohr 10 gezeigt.

Fig. 17 zeigt eine sechste Variante einer Ausführungsform eines Herzohroccluders 170 nach der Implantation im linken Herzohr 10. Der proximale Retentionsbereich 2 weist für die Ausführungsform nach Fig. 17 des Weiteren eine Vertiefung 8 auf, in der die Fassung 4 angeordnet ist, in welcher die Enden der Drähte bzw. Fäden des Geflechts 7 zusammenlaufen. Damit kann erreicht werden, dass im implantierten Zustand möglichst keinerlei Material des implantierten Occlusionsinstruments 1 über die Herzohrebene in die Blutbahn des Patienten hineinragen kann. Durch das Vorsehen einer derartigen Vertiefung 8 nach Fig. 17 im proximalen Retentionsbereich 2, kann des Weiteren ein Verbindungselement 9 am proximalen Retentionsbereich 2 angeordnet werden, ohne das eine mögliche Abwehrreaktion vom Körper des Patienten eintritt, da ein Blutkontakt mit dem in der Vertiefung 8 angeordneten Verbindungselement 9 wirksam verhindert wird. Das Verbindungselement 9 kann dahingehend ausgelegt sein, dass es mit einem Katheter in Eingriff bringbar ist.

Fig. 18 illustriert eine siebte Variante einer Ausführungsform eines Herzohroccluders 180, hergestellt aus einem Trichtergeflecht ohne Fassung, Hülse oder Klemme am distalen Ende 21 bzw. proximalen Ende 20. Der Herzohroccluder 180 kann aus einem einzigen Draht hergestellt sein.

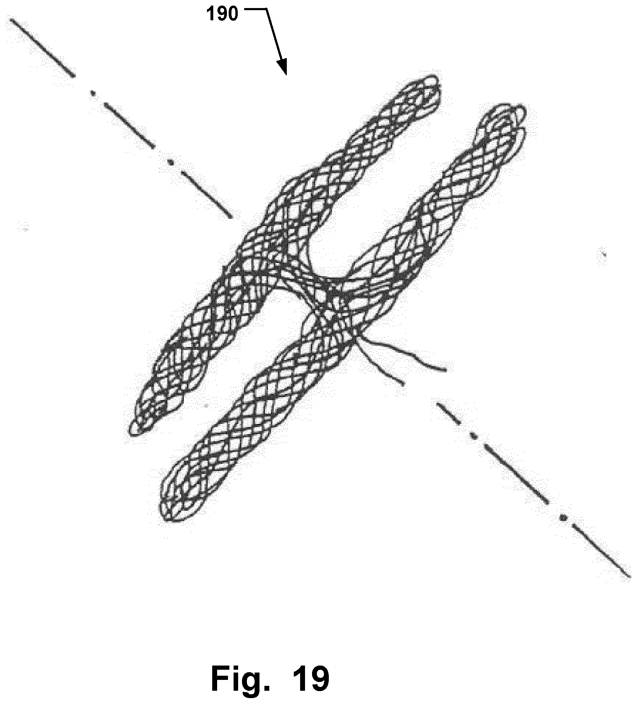

Fig. 19 bietet die Darstellung einer Seitenansicht eines PFO-Occluders 190 ohne Fassung, hergestellt aus einem Kugelgeflecht nach Fig. 7.

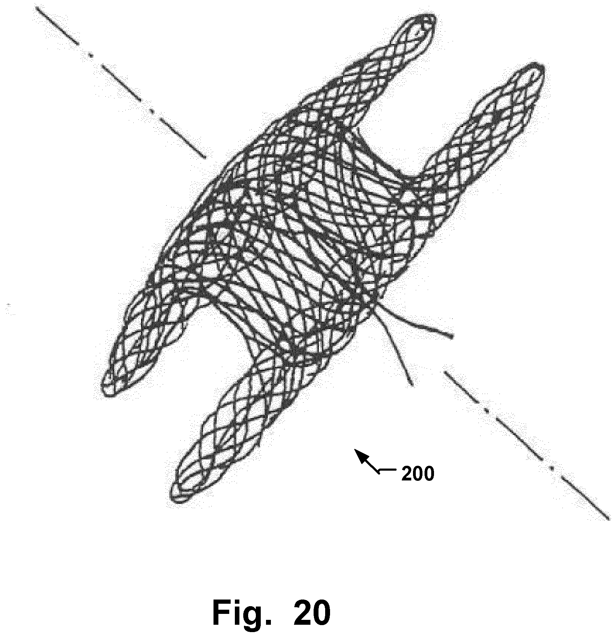

Eine Darstellung einer Seitenansicht eines ASD-Occluders 200 ohne Fassung, hergestellt nach einem Kugelgeflecht nach Fig. 7, zeigt Fig. 20. Der ASD-Occluders 200 kann aus einem einzigen Draht hergestellt sein.

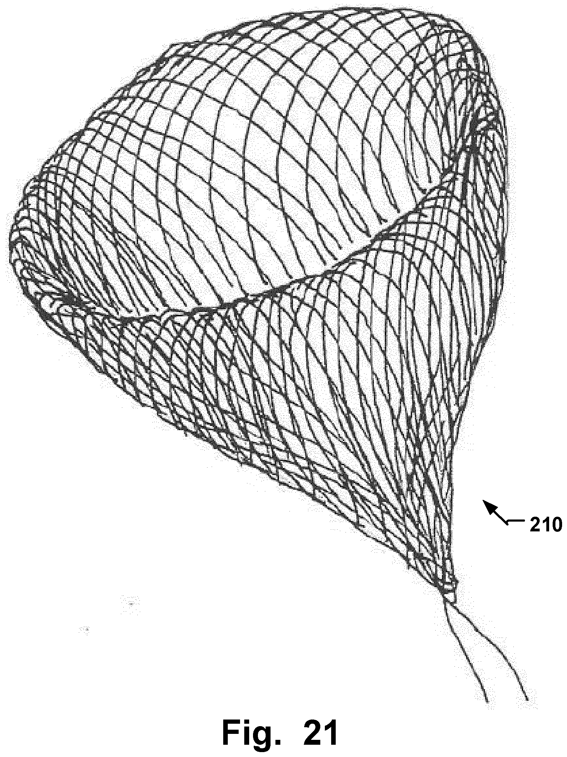

Ein Trichtergeflecht 210, hergestellt aus einem Draht nach dem 2-D-Flechtverfahren oder Flechtverfahren nach Occlutech, wird in Fig. 21 illustriert. Das Trichtergeflecht 210 kann aus einem einzigen Draht hergestellt sein.

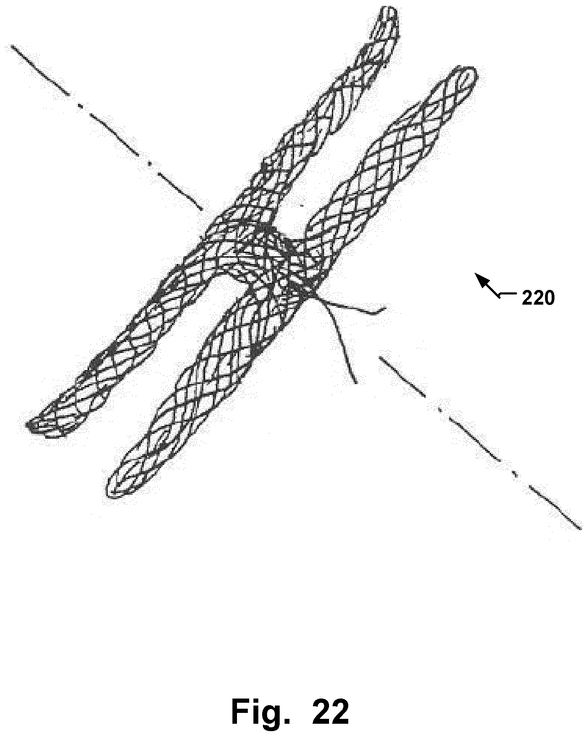

In einer Seitenansicht wird ein eines PFO-Occluder 220 ohne Fassung, in Fig. 22 gezeigt, hergestellt aus einem Draht nach dem Trichtergeflecht gemäß Fig. 21.

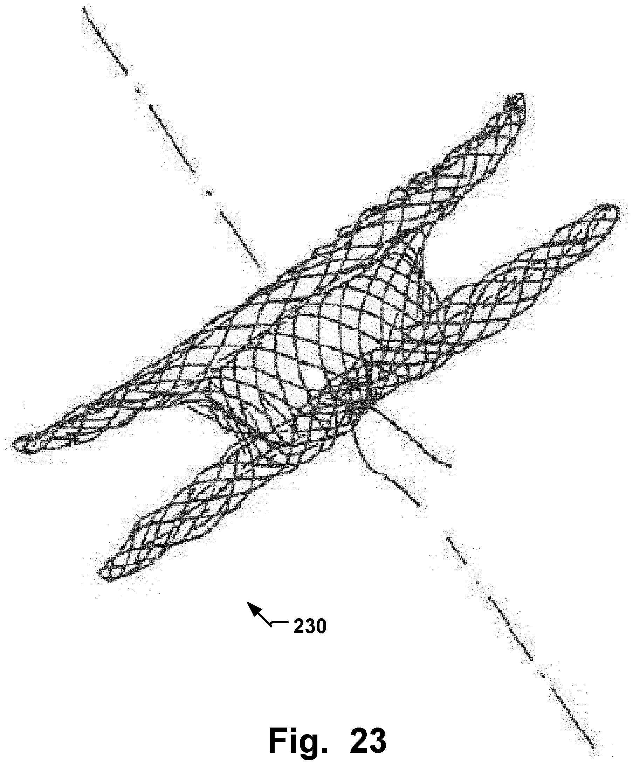

Fig. 23 ist eine Seitenansicht eines ASD-Occluders 230 ohne Fassung, hergestellt aus einem Draht nach dem Trichtergeflecht gemäß Fig. 21.

So ist in bevorzugter Weise für das Occlusionsinstrument vorgesehen, dass ein distaler Retentionsbereich mit einer Kugelform derart ausgelegt ist, dass er sich beim Expandieren des Occlusionsinstruments nach außen wölbt, um derart im implantierten Zustand mit den Innenwandungen des Herzohres zur Anlage zu kommen. Mit dieser bevorzugten Ausführungsform ist es demnach möglich, dass das selbst expandierbare Occlusionsinstrument mittels eines Einführ-Kathetersystems besonders tief in das zu verschließende Herzohr vorgeschoben werden kann. Der proximale Retentionsbereich, der in vorteilhafter Weise beispielsweise als proximales Schirmchen ausgebildet ist, wird anschließend, das heißt, nachdem das Occlusionsinstrument mithilfe des Kathetersystems in dem zu verschließenden Herzohr eingeschoben worden ist, zur Entfaltung gebracht und positioniert, wobei das Schirmchen am Rand der Öffnung des Herzohres zum Eingang des Herzohres anliegt. Gleichzeitig expandiert sich auch der distale Bereich des Occlusionsinstruments, das heißt der distale Kugelschirm, wobei beim Expansionsvorgang des distalen Kugelschirmchens der distale Retentionsbereich des Occlusionsinstruments weiter in das Herzohr hineingezogen wird und so über den Mittenbereich eine Zugkraft auf das proximale Schirmchen ausgeübt wird. Als eine direkte Folge hiervon wird das proximale Schirmchen bzw. der proximale Retentionsbereich unter einer permanenten Spannung am Eingang des Herzohres gehalten. Anders ausgedrückt bedeutet dies, dass mit der vorteilhaften Weiterentwicklung des selbst expandierbaren Occlusionsinstruments ein selbstpositionierendes und selbsthaltendes Occlusionsinstrument angegeben wird, wobei die Position des proximalen Schirmchens vorzugsweise bündig an der Öffnung des Herzohres mithilfe des sich selbstständig nach außen wölbenden distalen Kugelschirmchens gehalten wird.

Durch das einerseits flexible und andererseits kraftschlüssige Anliegen des Kugelbereiches mit einer relativ großen Oberfläche an der Innenwandung des Herzohres kann ferner erreicht werden, dass das eingesetzte Occlusionsinstrument deutlich schneller als bei den aus dem Stand der Technik bekannten Verschlusssystemen vollständig von körpereigenem Gewebe eingeschlossen werden kann.

Aus der Verwendung eines aus dünnen Drähten oder Fäden aufgebauten Geflechts als Ausgangsmaterial für das erfindungsgemäße Occlusionsinstrument leitet sich der weitere Vorteil ab, dass es eine langfristige mechanische Stabilität aufweist. Wie bereits angedeutet, kann das Auftreten von Brüchen in der Struktur oder anders artige Materialermüdung des eingesetzten Implantats weitgehend verhindert werden. Ferner besitzt das Geflecht eine ausreichende Steifigkeit.

Dadurch, dass am distalen Retentionsbereich auf eine Fassung zum Zusammenbündeln bzw. Zusammenfassen des Geflechts verzichtet werden kann, ragt auch keine Komponente des Occlusionsinstruments in das Herzohr weiter hinein, sodass das Risiko für Abwehrreaktionen des Körpers oder andere möglichen Komplikationen gering ist.

In einer besonders vorteilhaften Realisierung eines Occlusionsinstruments ist vorgesehen, dass prinzipiell auf jegliche Fassungen verzichtet werden kann, dies betrifft sowohl das proximale als auch distale Ende des Occlussionsinstrumentes. Dabei wurde ein besonderes Kugelgeflecht verwendet, welches aus nur einem Draht hergestellt wird. Grundlage sind die in der Literatur bekannten Verfahren des 2- und 3D-Flechtens, wobei hier die Flechtspindeln auf einer ebenen Fläche schachbrettartig angeordnet sind und untereinander mit beliebigen Richtungsänderungen solche Geflechtsgebilde hergestellt werden können. Eine weitere Herstellungsmöglichkeit von solchen Kugelgeflechten wurde bereits in der Occlutech Patentanmeldung Deutschland Nr. DE102006013770.1 Kugeloccluder beschrieben. Mit dieser Offenbarung ist es möglich, einzelne Drahtabschnitte so zu verflechten, dass Kugel-Occluder mit einer Fassung entstehen. DE102006013770.1 ist hiermit in seiner Gesamtheit durch Bezugsnahme beinhaltet.

Reduziert man die Anzahl der Drahtabschnitte auf genau einen Draht mit einer definierten Länge, lassen sich solche Kugelgeflechte ohne Fassungen herstellen. Die beiden übrig gebliebenen Drahtenden werden so miteinander verbunden, beispielsweise verschweißt, dass ein geschlossenes Kugelgeflecht entsteht. Damit kann letztlich bei einem entsprechenden Occlusionsinstrument sowohl distal als auch proximal auf eine Fassung verzichtet werden. Der große Vorteil bei einem solchen Herzohroccluder ist zum Beispiel, dass das proximale Ende des Occluders, welches im linken Herzohr platziert ist, kein Fassungselement trägt, sodass die Bildung von möglichen Tromben extrem reduziert werden kann. Die Drahtenden des einen Drahtes können hierbei an dem proximalen Ende des Herzohroccluders zusammengeführt sein, oder an einer anderen Position.

In den Patentanmeldungen von Occlutech Nr. PCT/EP 2005/012132 vom 11.11.2005, US 11/271,752 vom 14.11.2005 und Deutschlandanmeldung 102005053906.8 vom 11.11.2005 sind Vorrichtungen beschrieben, wo am distalen Ende der Vorrichtung zum Implantieren von solchen Occlusionsinstrumenten eine Schlaufe existiert, welche während des Implantationsvorganges die Verbindung zwischen dem Operationsbesteck und dem Occluder herstellt. Von der Dimensionierung können bekannte Einführschleusen mit einem Innendurchmesser von 6 bis 10 French verwendet werden.

Um zu erreichen, dass das Geflecht des Occlusionsinstruments mittels eines Umformungs- und Wärmebehandlungsverfahrens seine geeignete Formgebung erhalten kann, ist in einer besonders bevorzugten Ausführungsform vorgesehen, dass das Geflecht auf einem Formgedächtnismaterial, insbesondere Nitinol oder Kunststoff gebildet ist. Der Einsatz von Nitinol bei Occlusionsinstrumenten ist bekannt. Formgedächtnispolymere gehören zur Gruppe der intelligenten Polymere und sind Polymere, die einen Formgedächtniseffekt zeigen, d.h. unter Einwirkung eines äußeren Stimulus, wie z.B. einer Temperaturänderung, ihre äußere Form ändern können.

Dabei wird das Polymer zunächst durch konventionelle Verarbeitungsmethoden, wie etwa Spritzguss oder Extrusion, in seine permanente Form gebracht. Anschließend wird der Kunststoff deformiert und in der gewünschten temporären Form fixiert, was auch "Programmierung" genannt wird. Dieser Vorgang kann bei Polymeren einerseits so erfolgen, dass die Probe erwärmt, deformiert und dann abgekühlt wird. Andererseits kann das Polymer bzw. der Kunststoff auch bei niedriger Temperatur deformiert werden, was als "kaltes Verstrecken" bezeichnet wird. Damit ist die permanente Form gespeichert, während die temporäre Form aktuell vorliegt. Wird nun der Polymerformkörper auf eine Temperatur höher als die Schalttemperatur erwärmt, kommt es zum Auslösen des Formgedächtniseffekts und damit zur Wiederherstellung der gespeicherten permanenten Form. Durch Abkühlen der Probe bildet sich die temporäre Form nicht reversibel zurück, weshalb man von einem so genannten Ein-Weg-Formgedächtniseffekt spricht.

Im Vergleich zu bekannten Formgedächtnismaterialien wie z.B. der Formgedächtnislegierung Nitinol, einer äquiatomaren Legierung aus Nickel und Titan, sind Formgedächtnispolymere mit ihren Gedächtnisleistungen um ein vielfaches Überlegen. Dabei ist nur ein geringer Aufwand (Erwärmen bzw. Abkühlen) zur Programmierung der temporären Form bzw. zur Wiederherstellung der permanenten Form nötig. Darüber hinaus beträgt bei Nitinol die maximale Deformation zwischen permanenter und temporärer Form nur 8%. Formgedächtnispolymere weisen wesentlich höhere Verformbarkeit von bis zu 1.100% auf. Sämtliche vorgenannten Formgedächtnispolymere und Materialien werden mit der vorliegenden Offenbarung für die biomedizinische Anwendung des erfindungsgemäßen Occlusionsinstruments offenbart.

In einer vorteilhaften Weiterentwicklung der zuletzt genannten Ausführungsform des Occlusionsinstruments, bei welchem das Geflecht aus einem Formgedächtnismaterial gebildet wird, ist vorgesehen, dass das Material ein biologisch abbaubares Formgedächtnis-Polymermaterial aufweist. Insbesondere eignen sich synthetische, bioabbaubare Implantatmaterialien. Derartige abbaubare Werkstoffe bzw. Polymere enthalten unter physiologischen Bedingungen spaltbare Bindungen. Dabei spricht man von einer "Bioabbaubarkeit", wenn der Werkstoff unter Verlust der mechanischen Eigenschaft durch oder in einem biologischen System abgebaut wird. Die äußere Form und die Masse des Implantats bleibt während des Abbaus unter Umständen erhalten. Wird von einer Degradationszeit ohne zusätzliche quantifizierende Angaben gesprochen, so ist die Zeit, in der der vollständige Verlust der mechanischen Eigenschaft auftritt, gemeint. Unter biostabilen Werkstoffen versteht man solche, die in biologischen Systemen stabil sind und langfristig zumindest nur teilweise abgebaut werden.

Bei abbaubaren Polymeren unterscheidet man zwischen hydrolytisch und enzymatisch abbaubare Polymere. Der hydrolytische Abbau hat den Vorteil, dass die Abbaugeschwindigkeit unabhängig vom Ort der Implantation ist, da Wasser überall vorhanden ist. Demgegenüber ist die Konzentration an Enzymen lokal sehr unterschiedlich. Bei bioabbaubaren Polymeren oder Werkstoffen kann demnach der Abbau durch reine Hydrolyse, enzymatisch induzierte Reaktionen oder durch deren Kombination erfolgen. Typische hydrolysierbare chemische Bindungen sind Amid-, Ester- oder Acetal-Bindungen. Beim Abbau beobachtet man zwei Mechanismen. Beim Oberflächenabbau findet die Hydrolyse chemischer Bindungen ausschließlich an der Oberfläche statt. Aufgrund des hydrophoben Charakters erfolgt der Polymerbau schneller als die Diffusion von Wasser in das Innere des Materials. Diesen Mechanismus beobachtet man vor allem bei Poly(anhydride)n oder Poly(orthoester)n. Für die vor allem für den Formgedächtniseffekt bedeutsamen Poly(hydroxcarbonsäuren), wie Poly(milchsäure) oder Poly(glycosesäure) bzw. entsprechende Copolymere, erfolgt der Polymerabbau im gesamten Volumen. Der geschwindigkeitsbestimmende Schritt ist hierbei die hydrolytische Bindungsspaltung, da die Diffusion von Wasser in der eher hydrophylen Polymermatrix relativ schnell erfolgt. Für die Anwendung von bioabbaubaren Polymeren ist entscheidend, dass sie einerseits mit einer kontrollier- bzw. einstellbaren Geschwindigkeit abbauen und andererseits die Abbauprodukte nicht toxisch sind.

Sämtliche vorgenannten biologisch abbaubaren Formgedächtnis-Polymere werden einschließlich der weiteren möglichen Anwendungen aus dem Patent Deutschland-Anmeldung "medizinisches selbstexpandierendes Occlusionsinstrument" Nr. 10 2005 053 958.0 vom 11.11.2005, als PCT-Anmeldung PCT/EP2005/012 131 vom 11.11.2005 und als US-Anmeldung US11/271,750 vom 13.12.2005 mit ihrer Anwendung auf die in diesem Patent beschriebene bevorzugte Herzoccludervariante einschließlich der dargestellten, abgeleiteten Varianten, offenbart.

Gemäß Ausführungsformen des Occlusionsinstrumentes bestehen die Fäden 7a des Geflechts 7 aus einer Formgedächtnis-Polymerkomposition, sodass sich das Geflecht 7 unter Einwirken eines äußeren Stimulus von einer temporären Form zu einer permanenten Form verformt, wobei die temporäre Form in der ersten Formgebung des Geflechts 7 und die permanente Form in der zweiten Formgebung des Geflechts 7 vorliegt. Der äußere Stimulus kann eine festlegbare Schalttemperatur sein, beispielsweise im Bereich zwischen der Raumtemperatur und der Körpertemperatur eines Patienten.

Die Polymerkomposition gewisser Ausführungsformen weist polymere Schaltelemente auf, wobei unterhalb der festlegbaren Schalttemperatur die temporäre Form des Geflechts 7 mithilfe von charakteristischen Phasenübergängen der polymeren Schaltelemente stabilisiert wird.

Gemäß bestimmter Ausführungsformen weist die Polymerkomposition ein kristallines oder teilkristallines Polymernetzwerk mit kristallinen Schaltsegmenten auf, wobei die temporäre Form des Geflechts 7 durch ein Einfrieren der kristallinen Schaltsegmente beim Kristallisationsübergang fixiert und stabilisiert wird, wobei die Schalttemperatur durch die Kristallisationstemperatur bzw. Schalttemperatur der kristallinen Schaltsegmente bestimmt wird.

Gemäß bestimmter Ausführungsformen weist die Polymerkomposition ein amorphes Polymernetzwerk mit amorphen Schaltsegmenten auf, wobei die temporäre Form des Geflechts 7 durch ein Einfrieren der amorphen Schaltsegmente beim Glasübergang der Schaltsegmente fixiert und stabilisiert wird, wobei die Schalttemperatur durch die Glasübergangstemperatur der amorphen Schaltsegmente bestimmt wird.

Gemäß bestimmter Ausführungsformen weist die Polymerkomposition ein lineares, phasensegregiertes Multiblock-Copolymernetzwerk auf, welches in zumindest zwei verschiedenen Phasen vorliegen kann, wobei die erste Phase eine Hartsegment-bildende Phase ist, bei welcher im Polymer eine Vielzahl von Hartsegment-bildende Blöcke ausgebildet werden, welche zur physikalischen Vernetzung der Polymerstruktur dienen und die permanente Form des Geflechts 7 bestimmen und stabilisieren, und wobei die zweite Phase eine Schaltsegment-bildende Phase ist, bei welcher im Polymer eine Vielzahl von Schaltsegment-bildende Blöcke ausgebildet werden, die zur Fixierung der temporären Form des Geflechts 7 dienen, wobei die Übergangstemperatur der Schaltsegment-bildenden Phase zur Hartsegment-bildenden Phase die Schalttemperatur ist, und wobei oberhalb der Übergangstemperatur der Hartsegment-bildenden Phase die Formgebung des Geflechts 7 durch konventionelle Verfahren, insbesondere durch Spritzguss- oder Extrusionsverfahren, eingestellt werden kann.

Die Polymerkomposition kann thermoplastische Polyurethan-Elastomere mit einer Multiblockstruktur aufweisen, wobei die Hartsegment-bildende Phase durch eine Umsetzung von Diisocyanaten, insbesondere Methylen-bis(4-phenylisocyanat) oder Hexamethylendiisocyanat, mit Diolen, insbesondere 1,4-Butandiol, gebildet wird, und wobei sich die Schaltsegment-bildende Phase aus oligomeren Polyether- bzw. Polyesterdiolen, insbesondere ausgehend von OHterminierten Poly(tetrahydrofuran), Poly(ε-caprolaceton), Poly(ethylenadipat), Poly(ethylenglyocol) oder Poly(propylenglycol), ergibt.

Die Polymerkomposition kann auch phasensegregierte Diblockcopolymere mit einem amorphen A-Block und einem teilkristallinen B-Block aufweisen, wobei der Glasübergang vom amorphen A-Block die Hartsegment-bildende Phase bildet, und wobei die Schmelztemperatur des teilkristallinen B-Blocks als Schalttemperatur für den thermischen Formgedächtnis-Effekt dient. Die Polymerkomposition kann Polystyrol als amorphen A-Block und Poly(1,4-butadien) als teilkristallinen B-Block aufweisen.

Gemäß bestimmter Ausführungsformen weist die Polymerkomposition ein phasensegregiertes Triblockcopolymer mit einem teilkristallinen zentralen B-Block und mit zwei amorphen terminalen A-Blöcken auf, wobei die A-Blöcke das Hartsegment aufbauen, und wobei der B-Block die Schalttemperatur bestimmt. Die Polymerkomposition kann beispielsweise teilkristallines Poly(tetrahydrofuran) als zentralen B-Block und amorphes Poly(2-methyloxazolin) als terminale A-Blöcke aufweisen.

Gemäß bestimmter Ausführungsformen weist die Polymerkomposition Polynorbornen, Polyethylen-Nylon-6-Pfropfcopolymere und/oder vernetzte Poly(ethylen-covinylacetat)-Copolymere aufweist.

Gemäß bestimmter Ausführungsformen weist die Polymerkomposition ein kovalentes vernetztes Polymernetzwerk auf, welches durch Polymerisation, Polykondensation und/oder Polyaddition von difunktionellen Monomeren oder Makromeren unter Zusatz von tri- oder höherfunktionellen Vernetzern aufgebaut ist, wobei über eine geeignete Auswahl der Monomere, deren Funktionalität und dem Anteil am Vernetzer die chemischen, thermischen und mechanischen Eigenschaften des gebildeten Polymernetzwerkes gezielt einstellbar sind. Die Polymerkomposition kann hierbei beispielsweise ein kovalentes Polymernetzwerk sein, welches durch vernetzende Copolymerisation von Stearylacrylat und Methacrylsäure mit N,N'-Methylenbisacrylamid als Vernetzer aufgebaut ist, wobei der Formgedächtnis-Effekt der Polymerkomposition auf kristallisierenden Stearylseitenketten beruht.

Gemäß bestimmter Ausführungsformen weist die Polymerkomposition ein kovalent vernetztes Polymernetzwerk auf, welches durch eine nachträgliche Vernetzung von linearen oder verzweigten Polymeren gebildet wird. Die Vernetzung kann durch ionisierende Strahlung oder durch thermische Spaltung radikal bildender Gruppe ausgelöst werden.

Gemäß bestimmter Ausführungsformen weist die Polymerkomposition zumindest ein biologisch abbaubares Material auf. Die Polymerkomposition kann hierbei beispielsweise ein hydrolytisch abbaubares Polymer aufweisen, insbesondere Poly(hydroxycarbonsäuren) bzw. entsprechende Copolymere. Die Polymerkomposition weist besipielsweise enzymatisch abbaubare Polymere auf. Gemäß bestimmter Ausführungsformen weist die Polymerkomposition ein bioabbaubares thermoplastisches amorphes Polyurethan-Copolyester-Polymernetzwerk auf. Die Polymerkomposition kann ein bioabbaubares elastisches Polymernetzwerk aufweisen, welches durch Vernetzung von oligomeren Diolen mit Diisocyanat erhalten wird. Die Polymerkomposition kann hierbei auf der Basis von kovalenten Netzwerken ausgehend von Oligo(ε-caprolacton)dimethacrylat und Butylacrylat gebildet sein.

Besonders bevorzugt ist vorgesehen, dass sich das Geflecht des Occlusionsinstruments auf den Durchmesser eines bei dem minimal-invasiven Operationseingriff verwendeten Katheters verjüngen lässt. Der Vorteil dieser Ausführungsform ist darin zu sehen, dass die zur Implantation und Explantation zu verwendenden Kathetersysteme einen deutlich reduzierten Innendurchmesser aufweisen können, was die Manövrierbarkeit des zu implantierenden Occlusionsinstruments deutlich erhöht. Von daher kann die Positioniergenauigkeit des Instruments im Herzohr verbessert werden. Bei einem aus Nitinol bestehenden Occluder liegt der Innendurchmesser des zur Implantation oder Explantation verwendeten Katheters zwischen 8 bis 10 French, wohingegen bei der Verwendung von Occlusionsinstrumenten aus Polymerkunststoff der Innendurchmesser lediglich zwischen 6 bis 8 French liegen muss.

Schließlich ist besonders bevorzugt vorgesehen, dass das Occlusionsinstrument wenigstens eine Gewebeeinlage 120, 130 aufweist, die zum vollständigen Verschließen des Herzohrs im bzw. am proximalen Retentionsbereich angeordnet ist. Diese Gewebeeinlage dient dazu, die in den sich erweiternden Durchmessern des Occlusionsinstruments verbleibenden Zwischenräume nach dem Einsetzen und Expandieren des Instruments im Herzohr zu verschließen. Die Gewebeeinlage wird beispielsweise am distalen Retentionsbereich am Geflecht des Occlusionsinstruments derart befestigt, dass sie wie ein Tuch über den distalen Retentionsbereich gespannt werden kann. Der Vorteil dieser Konstruktion liegt darin, dass sich der Randsaum des distalen Retentionsbereiches bündig an die Herzohröffnung anlegt und weniger Fremdmaterial in den Körper des Patienten eingebracht wird. Die Gewebeeinlagen können beispielsweise aus Dacron (Polyethylenterephthalat) hergestellt sein. Selbstverständlich sind hier aber auch andere Materialien und andere Positionen der Gewebeeinlage im bzw. am Occlusionsinstrument denkbar einschließlich biologisch abbaubarer Materialien, welche vorstehend beschrieben wurden.

Die offenbarte Lösung weist eine Reihe wesentlicher Vorteile gegenüber der aus dem Stand der Technik bekannten und vorstehend genannten Occlusionsinstrumenten auf. Zum einen handelt es sich bei dem offenbarten Occluder um ein selbst expandierbares Instrument, was auf besonders einfache Weise mit beispielsweise einem geeigneten Einführkatheter implantierbar ist. Hierzu wird beispielsweise eine Vene im Bereich der Leiste des Patienten punktiert und das Einführkathetersystem bis an die Scheidewand des rechten Vorhofs vorgeführt. Mittels einer Punktion der Scheidewand des Vorhofs, was beispielsweise eine bekannte transseptale Punktion sein kann, wird der linke Vorhof des Herzens erreicht, sodass anschließend von der Leistenvene aus das Einführkathetersystem in das linke Herzohr eingebracht werden kann. Über das Einführkathetersystem kann anschließend das selbst expandierbare Occlusionsinstrument zum Verschließen des Herzohres eingebracht werden, die entsprechenden Schleusen haben einen verhältnismäßig kleinen Querschnitt, maximal 9 bis 10 F.

Das Occlusionsinstrument, welches während der Implantation im zusammengefalteten Zustand vorliegt, weist in bevorzugter Weise einen Durchmesser von 6 bis 10 French auf, sodass der Eingriff zum Verschließen des Herzohres minimal-invasiv ist.

Nachdem das zusammengefaltete Occlusionsinstrument mithilfe beispielsweise des Einführkatheters in dem zu verschließenden Herzohr positioniert ist, wird das Occlusionsinstrument von dem Katheter freigegeben, worauf sich dieses infolge seiner selbst expandierbaren Natur auseinander faltet und die mittels des bei Herstellung angewandten Umformungs- und Wärmebehandlungsverfahrens ausgeprägte Formgebung einnimmt. In diesem expandierten Zustand ist der rückseitige distale Retentionsbereich mit dem daran ausgebildeten Kugelbereich vollständig auseinander gefaltet und kommt an den Innenwandungen des zu verschließenden Herzohres zur Anlage. Dabei dient der distale Retentionsbereich mit dem daran ausgebildeten Kugelbereich zur Befestigung und Positionierung des expandierten Occlusionsinstruments in dem Herzohr. Der sich von dem distalen Retentionsbereich in Richtung Herzohröffnung erstreckende Mittenbereich sowie der am proximalen Ende des Mittenbereiches vorgesehene proximale Retentionsbereich füllen dabei den Öffnungsbereich des Herzohres nahezu vollständig aus, sodass das gesamte expandierte Occlusionsinstrument im eingesetzten Zustand als ein Verschlussstöpsel zum Verschließen des Herzohres dient. Auf diese Weise kann in besonders einfacher und minimal-invasiver Weise eine Trombusbildung mit dem Risiko eines Schlaganfalls erheblich reduziert werden.

Insbesondere dadurch, dass die Positionierung und Fixierung des Occlusionsinstruments mithilfe des an den Innenwandungen des Herzohres zur Anlage kommenden kugelförmigen Gebildes erreicht wird, kann bei dem Occlusionsinstrument auf Befestigungshäkchen oder andere Verankerungsmittel verzichtet werden, die üblicherweise bei derartigen Occlusionsinstrumenten zur Fixierung und Positionierung des Instrumentes im Gewebe verwendet werden. Dabei ist insbesondere zu beachten, dass infolge der äußerst dünnwandigen Ausgestaltung des Gewebes in der Umgebung des Herzohres die üblicherweise eingesetzten Befestigungshäkchen keine dauerhafte Befestigung und Positionierung des Occlusionsinstrumentes bereitstellen können. Mit der offenbarten Lösung und insbesondere durch einen am distalen Retentionsbereich ausgebildeten Kugelbereich kann die Problematik der Befestigung des Occlusionsinstruments an dem äußerst dünnwandigen und leicht zu verletzenden Herzohrgewebe mittels Häkchen umgangen werden.

Mit dem erfindungsgemäßen Verfahren wird eine besonders leicht zu realisierende Möglichkeit zur Herstellung des vorstehend beschriebenen Occlusionsinstruments angegeben. Dabei wird zunächst ein kugelförmiges Hohlgeflecht mittels beispielsweise einer Rundflechtmaschine ausgebildet. Es kann hier eine Technik zum Einsatz kommen, bei der das ausgebildete Geflecht am Ende der Flechtlänge, d.h. am späteren distalen Ende des Occlusionsinstruments, gebündelt wird und am Anfang der Flechtlänge, d.h. am späteren distalen Ende des Occlusionsinstruments, geschlossen bleibt. Damit ist es möglich, ein kugelförmiges Hohlgeflecht herzustellen, dessen gebündeltes Ende dem proximalen Ende des fertigen Occlusionsinstruments und dessen gegenüberliegendes geschlossene Ende dem distalen Ende des fertigen Occlusionsinstruments entspricht. Dadurch, dass zum Herstellen des Occlusionsinstruments ein an sich bekanntes Flechtverfahren verwendet werden kann, weist das gefertigte Occlusionsinstrument mechanische Eigenschaften hinsichtlich beispielsweise der Dehnung, Stabilität, Festigkeit etc. auf, die individuell an den späteren Einsatz des Occlusionsinstruments angepasst werden können. In vorteilhafter Weise können metallische Drähte, aber auch organische Fäden zu dem Geflecht verarbeitet werden.

Ebenso können Kugelgeflechte, wie beschrieben ohne Fassung zum Einsatz kommen.

Hinsichtlich des Verfahrens ist in bevorzugter Weise vorgesehen, dass der Verfahrensschritt des Ausformens der Retentionsbereiche und des Mittenbereichs einen Umformungs- und/oder Wärmebehandlungsschritt aufweist. Dieses ist insbesondere dann von Vorteil, wenn das ausgebildete kugelförmige Hohlgeflecht aus Nitinol oder aus einem anderen Material, insbesondere Polymer, mit Formgedächtnis- oder Memory-Effekt besteht. Für das offenbarte Occlusionsinstrument ist insbesondere vorgesehen, dass das Geflecht aus einem Formgedächtnispolymer ausgebildet ist, welches beispielsweise auf Polyanhydriden als Matrix oder auf Polyhydroxycarbonsäure basiert. Hierbei handelt es sich um synthetische abbaubare Materialien, die über einen thermisch induzierten Formgedächtniseffekt verfügen. Denkbar wären aber auch andere Formgedächtnispolymere, wie etwa Blockcopolymere wie sie beispielsweise im Sonderdruck angewandte Chemie 2002, 114, Seiten 2138 bis 2162 von A. Lendlein und S. Kelch beschrieben werden. Derartige Materialien lassen sich auf einfache Weise durch eine Kombination aus Umformungs- und Wärmebehandlungsschritten in eine entsprechende endgültige Form bringen. Ein fertig ausgebildeter Occluder lässt sich dann beispielsweise auf die Größe eines Katheters verjüngen. Nach dem Austritt aus dem Katheter entfaltet sich das Occlusionsinstrument dann selbstständig und nimmt wieder jene Formgebung an, die mittels des Umformungs- und/oder Wärmebehandlungsschritts dem kugel- bzw. trichterförmigen Hohlgeflecht des Occlusionsinstruments beim Herstellungsverfahren ausgeprägt wurde.

In bevorzugter Weise ist das kugelförmige Hohlgeflecht derart hergestellt, dass dünne Drähte oder Fäden, das fertige Geflecht begründen, beim Ausbilden des kugelförmigen Hohlgeflechts am distalen Ende des Geflechts untereinander verflochten werden. Dies stellt eine mögliche und besonders einfach zu realisierende Art und Weise dar, ein Occlusionsinstrument gemäß der vorliegenden Erfindung herzustellen, dessen distaler Retentionsbereich eine zum distalen Ende hin geschlossene Form aufweist. Selbstverständlich sind aber auch andere Herstellungsverfahren denkbar.

Zur Fertigung der Drahtgeflechte 7 wird beispielsweise auf das bereits erteilte Europapatent EP1651117B1 vom 24.01.2007 und die Occlutech-Patentanmeldung Deutschland Nr. 102006013770.1 Kugeloccluder vom 24.03.2006 verwiesen.

Es sei darauf hingewiesen, dass die Ausführung der Erfindung nicht auf das in den Figuren beschriebene Ausführungsbeispiel beschränkt ist, sondern auch in einer Vielzahl von Varianten möglich ist. Die Erfindung ist alleine durch die Patentansprüche definiert.

### Bezugszeichenliste

1 Occlusionsinstrument
2 proximaler Retentionsbereich
3 distaler Retentionsbereich
4 Fassung
5 Mittenbereich
6 Kugelbereich
7 Geflecht
7a Faden des Geflechts 7
8 Vertiefung
9 Verbindungselement
10 Herzohr
11 Herzohröffnung
12 Wandung der Herzohröffnung
13 Herzohrwand
20 proximales Ende
21 distales Ende
30 kugelförmiges hohles Kugelgeflecht
40 ballonförmiges hohles Kugelgeflecht
50 Herzohroccluder ohne Fassung
70 Kugelgeflecht aus einem Drahtstück ohne Fassung
80 zweite Variante einer Ausführungsform eines Herzohroccluders
100 Dritte Variante einer Ausführungsform des Herzohroccluders
120 Gewebeeinlage
130 Gewebeeinlage
140 Vierte Variante einer Ausführungsform eines Herzohroccluders
150 Fünfte Variante einer Ausführungsform eines Herzohroccluders
153 Zwischen-Retentionsbereich
155 Hinterer Mittenbereich
170 Sechste Variante einer Ausführungsform eines Herzohroccluders
180 Siebte Variante einer Ausführungsform eines Herzohroccluders
190 PFO-Occluder 190 ohne Fassung
200 ASD-Occluder 200 ohne Fassung
210 Trichtergeflecht
220 PFO-Occluders
230 ASD-Occluders

## Patentansprüche

1. Occlusionsinstrument (100) bestehend aus einem einzigen Geflecht aus mindestens einem Draht oder Faden, wobei das Geflecht ein Hohlgeflecht ist und mit einem Umformungs- und/oder Wärmebehandlungsverfahren eine geeignete Formgebung erhalten hat, und selbst expandierbar ist, und das Occlusionsinstrument zur sicheren Verankerung in einem Herzohr (10) des linken oder rechten Vorhofes eines Herzens konfiguriert ist, wobei das Geflecht nach Formgebung umfasst:
einen proximalen Retentionsbereich (2) mit einer proximalen Fassung (9) am proximalen Ende (20) des Occlusionsinstrument in welcher die Drahtenden zusammenlaufen,
einen distalen Retentionsbereich (3), sowie
einen taillierten, zylindrischen Mittenbereich (5) zur flexiblen Beweglichkeit des distalen Retentionsbereiches (3) gegenüber dem proximalen Retentionsbereich (2); und wobei das Geflecht des Occlusionsinstrumentes (1) ein geschlossenes distales Ende (21) ohne Fassung aufweist und wenigstens teilweise kugelförmig und hohl ausgeformt ist.

2. Occlusionsinstrument gemäß Anspruch 1, wobei der genannte Mittenbereich eine für die Anwendung vorab festgelegte Länge hat, um das Verschließen der Herzohröffnung zu gewährleisten.

3. Occlusionsinstrument gemäß Anspruch 1 bis 2, wobei der proximale Retentionsbereich eine Vertiefung aufweist, in der eine Fassung angeordnet ist, in welcher die Enden der Drähte bzw. Fäden des Geflechts zusammenlaufen.

4. Occlusionsinstrument gemäß einem der vorstehenden Ansprüche, wobei das Geflecht ein Kugel-Drahtgeflecht ist.

5. Occlusionsinstrument gemäß einem der vorstehenden Ansprüche 1-4, wobei das Occlusionsinstrument in expandiertem Zustand mit dem distalen Retentionsbereich an den Innenwandungen eines zu verschließenden Herzohres zur Anlage kommt.

6. Occlusionsinstrument gemäß einem der vorstehenden Ansprüche 1-5, wobei der Mittenbereich sowie der am proximalen Ende des Mittenbereiches vorgesehene proximale Retentionsbereich konfiguriert sind, um den Öffnungsbereich des Herzohres nahezu vollständig auszufüllen, sodass das gesamte expandierte Occlusionsinstrument im eingesetzten Zustand als ein Verschlussstöpsel zum Verschließen des Herzohres dient.

7. Occlusionsinstrument gemäß einem der vorstehenden Ansprüche 1-6, wobei der mindestens eine Draht oder Faden aus Nitinol besteht.

8. Occlusionsinstrument gemäß einem der vorstehenden Ansprüche 1-7, wobei das Occlusionsinstrument keine Befestigungshäkchen oder andere Verankerungsmittel umfasst.

9. Verfahren zur Herstellung eines Occlusionsinstrumentes zum Verschließen einer Öffnung im Herzen gemäß einem der vorstehenden Ansprüche 1-8, wobei ein Hohlgeflecht derart hergestellt wird, dass mindestens ein dünner Draht oder Faden, welcher das fertige Geflecht begründet, beim Ausbilden des Hohlgeflechts am distalen Ende des Geflechts untereinander verflochten werden, sodass das Hohlgeflecht eine zum distalen Ende hin geschlossene Form ohne Fassung aufweist, umfassend
in einem Umformungs- und/oder Wärmebehandlungsschritt Ausformen des Occlusionsinstrumentes aus dem Hohlgeflecht, umfassend Ausformen eines proximalen Retentionsbereiches mit einer proximalen Fassung am proximalen Ende des Hohlgeflechtes in welcher die Drahtenden zusammenlaufen,
eines distalen Retentionsbereiches, sowie
eines taillierten, zylindrischen Mittenbereiches zur flexiblen Beweglichkeit des distalen Retentionsbereiches gegenüber dem proximalen Retentionsbereich, wobei
das Hohlgeflecht wenigstens teilweise kugelförmig und hohl ausgeformt ist und aus einem Material mit Formgedächtnis- oder Memory-Effekt besteht.

## Claims

1. An occlusion device (100) composed of a single braid of at least one thread or wire wherein the braid is a hollow braid which has been given a suitable shape during a moulding and/or heat treatment procedure and which is expandable and wherein the occlusion device is configured to allow secure anchoring in an auricle (10) of the left or right atrium of a heart wherein the braid, after moulding, comprises:
a proximal retention zone (2) with a proximal hub (9) on the proximal end (20) of the occlusion device in which the thread ends converge,
a distal retention zone (3) as well as
a cylindrical central zone (5) for the flexible mobility of the distal retention zone (3) in relation to the proximal retention zone (2) and wherein the braid of the occlusion device (1) has a closed distal end (21) without hub which is at least partially spherical and hollow.

2. An occlusion device according to claim 1 wherein the said central portion is of a predefined length for the application to ensure occlusion of the auricle orifice.

3. An occlusion device according to one of the claims 1 to 2 in which the proximal retention zone shows a recess in which there is a hub in which the ends of the wires or threads of the braid converge.

4. An occlusion device according to one of the above claims in which the braid is a spherical wire braid.

5. An occlusion device according to one of the claims 1 to 4 above in which the occlusion device in its expanded state rests with its distal retention zone against the inner walls of an auricle to be closed.

6. An occlusion device according to one of the claims 1 to 5 above in which the central zone as well as the retention zone provided on the proximal end of this central zone are configured to occupy almost the entire opening area of the auricle so that the entire implanted and expanded occlusion device serves as a closure plug for the auricle.

7. An occlusion device according to one of the claims 1 to 6 above in which the wires or threads are made of nitinol.

8. An occlusion device according to one of the claims 1 to 7 above in which the occlusion device does not comprise any fixing hooks or other anchoring means.

9. A manufacturing process of an occlusion device for closing an orifice in the heart according to one of the claims 1 to 8 above wherein the hollow braid is manufactured such that at least one wire or thread at the base of the finished braid is intertwined on the distal end of the braid during the formation of the hollow braid so that the hollow braid has a closed shape without hub towards the distal end comprising,
during a step of the moulding and/or heat treatment procedure, moulding of the occlusion device from the hollow braid with moulding of a proximal retention zone with a proximal hub on the proximal end of the hollow braid in which the thread ends converge,
a distal retention zone as well as
a cut, cylindrical central zone for the flexible mobility of the distal retention zone in relation to a proximal retention zone in which the hollow braid is at least partially spherical and hollow and consists of a material with a shape memory or memory effect.

## Revendications

1. Dispositif d'occlusion (100) composé d'un tressage unique d'au moins un fil ou un fil métallique dans lequel le tressage est un tressage creux qui a reçu une forme appropriée lors d'une procédure de moulage et/ou de traitement thermique et qui est expansible et dans lequel le dispositif d'occlusion est configuré pour permettre un ancrage en toute sécurité dans une auricule (10) de l'atrium gauche ou droite d'un cœur dans lequel le tressage, après moulage, comprend :
une zone de rétention proximale (2) avec un moyeu proximal (9) sur l'extrémité proximale (20) du dispositif d'occlusion dans lequel convergent les extrémités de fils,
une zone de rétention distale (3) ainsi
qu'une zone centrale cylindrique (5) pour la mobilité flexible de la zone de rétention distale (3) par rapport à la zone de rétention proximale (2) et dans lequel le tressage du dispositif d'occlusion (1) présente une extrémité distale (21) fermée sans moyeu qui est au moins partiellement sphérique et creuse.

2. Dispositif d'occlusion selon la revendication 1 dans lequel ladite partie centrale est d'une longueur prédéfinie pour l'application afin d'assurer l'occlusion de l'orifice de l'auricule.

3. Dispositif d'occlusion selon une des revendications 1 à 2 dans lequel la zone de rétention proximale montre un creux dans lequel se trouve un moyeu dans lequel les extrémités des fils métalliques ou des fils du tressage convergent.

4. Dispositif d'occlusion selon l'une des revendications ci-avant dans lequel le tressage est un tressage sphérique en fil métallique.

5. Dispositif d'occlusion selon l'une des revendications 1 à 4 ci-avant dans lequel le dispositif d'occlusion dans son état expansé se met en appui avec sa zone de rétention distale contre les parois internes d'une auricule à fermer.

6. Dispositif d'occlusion selon l'une des revendications 1 à 5 ci-avant où la zone centrale ainsi que la zone de rétention prévue sur l'extrémité proximale de cette zone centrale sont configurées pour occuper la quasi-totalité de la zone d'ouverture de l'auricule de sorte que l'ensemble du dispositif d'occlusion implanté et expansé serve de bouchon de fermeture de l'auricule.

7. Dispositif d'occlusion selon l'une des revendications 1 à 6 ci-avant dans lequel le ou les fils métalliques ou les fils sont en nitinol.

8. Dispositif d'occlusion selon l'une des revendications 1 à 7 ci-avant dans lequel le dispositif d'occlusion ne comprend ni crochets de fixation ni autre moyen d'ancrage.

9. Procédé de fabrication d'un dispositif d'occlusion pour fermer un orifice dans le cœur selon l'une des revendications 1 à 8 ci-avant dans lequel le tressage creux est fabriqué de telle sorte qu'au moins un fil métallique ou fil à la base du tressage fini est entrelacé sur l'extrémité distale du tressage lors de la formation du tressage creux de sorte que le tressage creux présente une forme fermée sans moyeu vers l'extrémité distale comprenant,
lors d'une étape de la procédure de moulage et/ou du traitement thermique, le moulage du dispositif d'occlusion à partir du tressage creux avec le moulage d'une zone de rétention proximale avec un moyeu proximal sur l'extrémité proximale du tressage creux dans lequel convergent les extrémités de fil,
une zone de rétention distale ainsi
qu'une zone centrale taillée, cylindrique pour la mobilité flexible de la zone de rétention distale par rapport à une zone de rétention proximale dans laquelle le tressage creux est au moins partiellement sphérique et creux et est constitué d'un matériau ayant une mémoire de forme ou un effet de mémoire.
